# EUROPEAN PATENT SPECIFICATION

(11) **EP 0 697 814 B1**
(45) Date of publication and mention of the grant of the patent: **30.07.2003**
(21) Application number: 94917274.6
(22) Date of filing: 28.04.1994
(51) Int. Cl.: A01N 43/90, A61K 9/00

(54) **POUR-ON FORMULATIONS CONTAININNG POLYMERIC MATERIAL, GLYCOLS AND GLYCERIDES**
POUR-ON FORMULIERUNG ENTHALTEND POLYMERISCHE MATERIAL, GLYKOLE UND GLYZERIDEN
FORMULATIONS DESTINEES AU TRAITEMENT CUTANE PAR DEVERSEMENT, CONTENANT UNE SUBSTANCE POLYMERE, DES GLYCOLS ET DES GLYCERIDES

(30) Priority: 10.05.1993 US 59787; 10.05.1993 US 59699
(43) Date of publication of application: 28.02.1996
(73) Proprietor: Merck & Co., Inc., Rahway New Jersey 07065-0900 (US)
(72) Inventor: CHOI, Hoo-Kyun, Blue Bell, PA 19422 (US); WILLIAMS, James, B., Lansdale, PA 19446 (US)
(74) Representative: Hiscock, Ian James
(86) International application number: US9404664
(87) International publication number: WO94026113

(56) References cited:
- EP-A- 0 045 655
- EP-A- 0 051 786
- EP-A- 0 120 286
- EP-A- 0 136 033
- EP-A- 0 137 627
- EP-A- 0 146 414
- EP-A- 0 193 347
- EP-A- 0 249 409
- EP-A- 0 329 460
- EP-A- 0 432 494
- FR-A- 2 599 220

## Description

### BACKGROUND OF THE INVENTION

The avermectin series of compounds are potent anthelmintic and antiparasitic agents against internal and external parasites. The natural product avermectins are disclosed in U.S. 4,310,519 to Albers-Schonberg et al., and the 22,23-dihydroavermectin compounds are disclosed in Chabala et al., U.S. 4,199,569. Administration of the avermectin compounds occur orally, parenterally or topically.

However, the conventional topical formulations do not provide acceptable efficacy against ectoparasites, especially against Chorioptes, fleas and ticks. Often times these formulations fail due to the lack of extended efficacy. The animals are readily reinfested by fleas, ticks and the like after treatment with the above-noted formulations simply by returning to a flea infested environment. Further, topical formulations of currently available medicinal agents have not demonstrated efficacy against endoparasites, such as heartworms and nematodes.

It is known in the pet care industry that sustained release of an insecticide is obtained by incorporation of the insecticide into a polymeric system. However, conventional polymer based formulations rely on the vaporization of the active compounds, which means this type of system may not be used for non-evaporable drugs. See U.S. Pat. Nos. 3,852,416 and 4,172,904. Additionally, conventional formulations of current medicinal agents require a withdrawal period of a few weeks after application of the active compound before any milk can be withdrawn from dairy animals for human consumption.

### SUMMARY OF THE INVENTION

This invention is concerned with avermectin topical pour-on formulations which effectively eliminate both ectoparasites, especially Chorioptes, fleas and ticks, and endoparasites, especially heartworms and nematodes, of animals such as cattle, swine, etc for an extended period up to a full four weeks, particularly household pets such as cats and dogs. The instant formulations also unexpectedly provides a zero milk withdrawal time for topically applied antiparasitic agents with regard to dairy animals. The formulations are prepared using solvents such as water, alcohols such as ethanol, methanol, isopropanol and the like, propylene glycol esters, glycerides, or their derivatives as the carrier.

The formulations can contain in addition to the active avermectin ingredient and solvent, a polymer such as polyvinylpyrrolidone. The drug is bound to the skin with the aid of the polymer which remains on the skin surface after the solvents have evaporated following application. Thus it is an object of this invention to describe such ectoparasitic and endoparasitic efficacy. Another object is to describe the avermectin compounds which may be employed in the formulation. A still further object is to describe how the concentration of the active compound in the milk of dairy animals is maintained below a concentration level that provides for a zero withdrawal period for human consumption. A still further object is to describe how extended efficacy against ticks, fleas and heartworms is obtained. Additional objects will become apparent after a reading of the following description.

### DESCRIPTION OF THE INVENTION

This invention consists of a topical formulation of a glyceride, glycol, or a derivative thereof and an avennectin compound which has been found to effectively eliminate both ectoparasites and endoparasites.The formulation also contains, in addition to the glyceride, glycol or derivative thereof and avermectin, an antioxidant such as BHA, BHT and the like, and can optionally contain additives such as Crodamol CAP™, glycerol formal, Tween 80™ and the like, a solvent mixture of water and/or solvents with relative high vapor pressure such as ethanol, methanol, isopropanol and the like, and a polymeric material such as polyvinyl pyrrolidone, polyvinyl alcohol and the like.

The avermectin compounds used in the instant formulations have the following general structure: 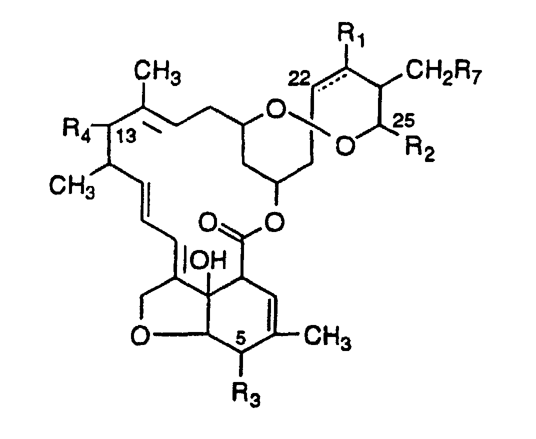 where the broken line indicates a single or a double bond at the 22,23-positions;
R₁ is hydrogen or hydroxy provided that R₁ is present only when the broken line indicates a single bond;
R₂ is alkyl of from 1 to 6 carbon atoms or alkenyl of from 3 to 6 carbon atoms or cycloalkyl of from 3 to 8 carbon atoms;
R₃ is hydroxy, methoxy or = NOR₅ where R₅ is hydrogen or lower alkyl;
R₇ is hydrogen, hydroxy, or lower alkyl; and
R₄ is hydrogen, hydroxy, poly C(1-6) alkoxy or 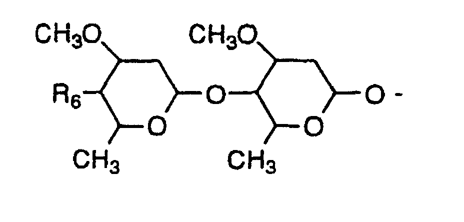 where R₆ is hydroxy, amino, mono-or di-C₁ to C₆ alkylamino or C₁ to C₆ alkanoylamino.

The term "loweralkyl" when used in the instant application is intended to represent those alkyl groups either straight or branched chain which have from 1-5 carbon atoms. Examples of such alkyl groups are methyl, ethyl, propyl, iso-propyl, butyl, sec-butyl, pentyl, and the like.

The term "loweralkanoyl" is intended to include those alkanoyl groups containing from one to five carbon atoms in either a straight or branched chain. Examples of such alkanoyl groups are formyl, acetyl, propenyl, butyryl, valeryl, and the like.

The term "halogen" is intended to include those halogen atoms fluorine, chlorine, bromine and iodine.

The term "polyalkoxy" is intended to include methoxymethoxy, 2-methoxyethoxy, (2-methoxyethoxy)-methoxy, [2-(2-methoxyethoxy)ethoxy]methoxy; and the like.

A related family of natural products also useful in the present invention is known as the milbemycins. The milbemycins have the same macrocyclic ring structures as the avermectins but have no substitution at position 13 (R₄ = hydrogen) and have a methyl or ethyl group at position 25 (R₂ = methyl or ethyl rather than isopropyl or sec-butyl as in the avermectins). The milbemycins and the fermentation conditions used to prepare them are described in U.S. Pat. No. 3,950,360. Closely related 13-deoxyavennectin aglycones are prepared by chemical modification of the natural avermectins and have been described in U.S. Pat. No. 4,173,571.

One preferred embodiment (E1) of this invention consists of a topical pour-on formulation of a gylceride, glycol, or a derivative thereof as a carrier and an avermectin compound which has been found to effectively eliminate both ectoparasites, especially Chorioptes, and endoparasites, while simultaneously maintaining the concentration of the active compound in the milk of dairy animals below an adequate concentration period for human consumption to provide a zero milk withdrawal time for topically applied endectocides [ milk concentration of 4"-acetylamino-4"-deoxyavermectin B1 (L-653,648) for zero milk withdrawal is 48 ng/ml].

The carriers are oleyl alcohol, propylene glycol and its esters such as propylene dicaprylate/dicaprate, propylene glycol laurate, and the like, glycol ethers such as diethylene glycol monoethyl ether, diethylene glycol monobutyl ether, diethylene glycol diethyl ether and the like, and glycerides such as PEG-6 caprylic/capric triglyceride, caprylic/capric diglyceryl succinate, polyglycolysed glycerides, and the like, preferrably propylene caprylate/caprate or caprylate caprate glyceride, and is available under such brand names as Miglyol 810, 812, 818, 829 and 840, Softigen and Labrasol®. The (/) in propylene dicaprylate/dicaprate and PEG-6 caprylic/capric triglycerides indicates a mixture of the two components in a ratio of 65-80/15-30.

The above carriers impart to the formulation good penetration and spreadability of the active compound even at cold temperatures.

The preferred avermectin compounds of E1 have the following structural formula: 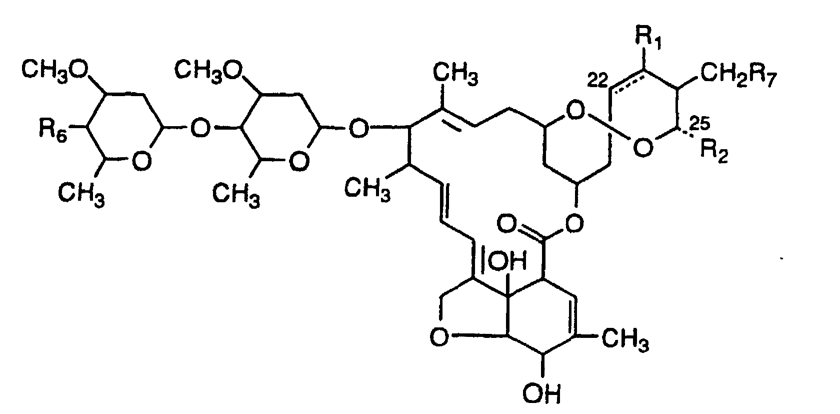 wherein the broken line represents a single bond; R₁ is hydrogen; R₂ is isopropyl of sec-butyl; R₆ is hydroxy, amino, mono-or di-C₁ to C₆ alkyl-amino or C₁ to C₆ alkanoylamino; and R₇ is hydrogen, hydroxy, or loweralkyl.

Examples of preferred compounds of the instant E1 formulation are:
4"-keto avermectin B1;
4"-keto avermectin B2;
4"-keto-22,23-dihydro avermectin B1;
4"-keto-22,23-dihydro avermectin B2;
4"-deoxy-4"-amino avermectin B1;
4"-deoxy-4"-amino avermectin B2;
4"-deoxy-4"-amino-22,23-dihydro avermectin B1;
4"-deoxy-4"-amino-22,23-dihydro avermectin B2;
4"-deoxy-4"-acetylamino avermectin B1;
4"-deoxy-4"-acetylamino avermectin B2;
4"-deoxy-4"-acetylamino-22,23-dihydro avermectin B1;
4"-deoxy-4"-acetylamino-22,23-dihydro avermectin B2;
4"-deoxy-4"-dimethylamino avermectin B1;
4"-deoxy-4"-dimethylamino avermectin B2;
4"-deoxy-4"-dimethylamino-22,23-dihydro avennectin B1;
4"-deoxy-4"-dimethylamino-22,23-dihydro avermectin B2;
4"-deoxy-4"-p-chloro benzenesulfonylamino-22,23-dihydro avermectin B1;
4"-deoxy-4"-p-chloro benzenesulfonylamino-22,23-dihydro avermectin B2;
4"-deoxy-4"-(2-methylbenzenesulfonylamino)-avermectin B1;
4"-deoxy-4"-(2-methylbenzenesulfonylamino)-avermectin B2.

The "b" compounds, those with a 25-iso-propyl group, are not necessarily separated from the corresponding "a" compound with a 25-sec-butyl group and the compounds are generally isolated as mixtures of the two compounds, consisting of at least 80% of the sec-butyl compound and no more than 20% of the iso-propyl compound. Thus references in the instant application to "a" compounds such as Bla, Ala, and the like, are construed to actually contain a certain proportion of the corresponding "b" compound. Alternatively, this representation of a mixture is sometimes done by referring to the B1 or B2 compounds or by separating the "a" compound from the "b" compound by a slash (/) such as B1a/B1b, B2a/B2b and the like. Additionally, the products of synthetic procedures such as racemization or epimerization, procedures known to those skilled in the art, can be a mixture of stereoisomers. In particular, the stereoisomers at the 13- and 23-positions may be oriented either α- or β- representing such groups being below or above the general plane of the molecule, respectively. In each case, and at other positions in the molecule, both the α- and β- configurations are intended to be included within the ambit of this invention.

In the topical forms of the avermectin formulation it has not been possible to provide a formulation which provides an acceptable efficacy against ectoparasites, especially Chorioptes. Additionally, currently available topical formulations do not provide a zero milk withdrawal time with the application of endectocides which thus precludes the use of such compounds on milk producing animals.

E1 of the instant invention gives the advantages of a pour-on topical formulation which provides the animal with effective treatment and protection against endoparasites and ectoparasites, especially Chorioptes and at the same time maintains the concentration of the active compound in the milk of dairy animals below a safe concentration for human consumption. Additional advantages of this invention are that the formulation is non-flammable, it is not readily washable by rain, it has good spreadability and cold temperature usage and has good compatibility with currently available dosing devices.

E1 can contain the avermectin compound and the glycol or glyceride carrier as the only ingredients. The formulations will generally be prepared to administer a safe and effective amount from 0.005 to 10% by weight of the avermectin component, most preferrably from 0.01 to 5% by weight. Most preferably a formulation containing about 0.5% of the avermectin is employed. At a preferred dose volume of about 5 ml to treat 50 kg of animal body weight the formulation contains from 1.0 to 50 mg of avermectin compound per ml of solution. The glycol or glyceride carrier is added to the formulation from 40 to 100% q.s.v/v (q.s. = quantity necessary to arrive at a total of 100% v/v).

The most preferred formulation for E1 contains in addition to the glycol, glyceride, or derivatives thereof and avermectin compound, an antioxidant such as propyl gallate, BHA (butylated hydroxy anisole), BHT (butylated hydroxy toluene) monothioglycerol and the like, preferrably BHT. The anti-oxidants are generally added to the formulation at rates of from 0.005 to 1.0% (w/v). Additives such as Crodamol™ CAP, glycerol formal, Tween 80 propylene glycol and the like, preferrably Crodamol™ CAP, may also be used. The additives are generally added to the formulation at volumes of up to 60% of the volume of glycol or glyceride carrier, preferrably up to 40% of the volume of carrier.

E1 is prepared by dissolving the avermectin compound in approximately 50-100% of the intended volume of the above mentioned carriers and then adjusting the volume to 100% by the addition of the final volume of the carrier or additive. The anti-oxidant and additive may be combined with the above mentioned carriers prior to mixing the avermectin or added as the final volume of solvent.

The following example is provided in order that the E1 embodiment of the invention might be more fully understood.

### EXAMPLE OF THE INVENTION

The formulations of this invention depend upon the particular avermectin compound and treatment. The avermectin is dissolved in approximately 50% of the glycol or glyceride carrier. When dissolved, the antioxidant and/or additive are optionally added and the volume adjusted to 100% with the final volume of glycol or glyceride carrier. The solution is mixed until it becomes homogeneous.
Generally, mixing at room temperature (15-25°C) is adequate however, if necessary, warming up to 50°C may be helpful. The following are nonlimiting examples of the composition of the present invention, which are conventionally formulated by mixing all components as stated above.

| Composition I | |
|---|---|
| 4"-acetylamino-4"-deoxyavermectin B1 | 0.5 % w/v |
| BHT | 0.01% w/v |
| Crodamol™ CAP | 10.0 % v/v |
| Miglyol 840 (q.s.) | 100.0 % v/v |

| Composition II | |
|---|---|
| 4"-acetylamino-4"-deoxyavermectin B1 | 0.5 % w/v |
| BHT | 0.01% w/v |
| Miglyol™ 840 (q.s.) | 100.0 % v/v |

| Composition III | |
|---|---|
| 4"-acetylamino-4"-deoxyavermectin B1 | 0.5 % w/v |
| BHT | 0.01% w/v |
| Isopropyl Myristate | 10.0 % v/v |
| Miglyol™ 840 (q.s.) | 100.0 % v/v |

| Composition IV | |
|---|---|
| 4"-acetylamino-4"-deoxyavermectin B1 | 0.5 % w/v |
| Triacetin | 50.0 % v/v |
| Miglyol™ 840 (q.s.) | 100.0 % v/v |

| Composition V | |
|---|---|
| 4"-acetylamino-4"-deoxyavermectin B1 | 0.5 % w/v |
| Softigen™ 767 | 65.0 % v/v |
| Miglyol™ 840 | 25.0% v/v |
| Ethanol (q.s.) | 100.0 % v/v |

| Composition VI | |
|---|---|
| 4"-acetylamino-4"-deoxyavermectin | 0.5 % w/v |
| Softigen™ 767 | 65.0 % v/v |
| Isopropanol (q.s.) | 100.0 % v/v |

| Composition VII | |
|---|---|
| 4"-acetylamino-4"-deoxyavermectin B1 | 0.5 % w/v |
| BHT | 0.01 % w/v |
| Dowanol™ DB (q.s.) | 100.00% v/v |

Crodamol™ CAP is a tradename mixture of isopropyl myristate, cetyl octanoate and stearyl octanoate and Dowanol™ DB is a tradename for diethylene glycol butyl ether.

### E1 EXAMPLE II

The data below are results indicating the avermectin concentration (ng/ml) in the milk of lactating cows after topical application with some of the above formulations and that the avermectin concentration is maintained below 48 ng/ml which is the milk concentration of avermectin required for a zero milk withdrawal.

| 4"-ACETYLAMINO-4"-DEOXYAVERMECTIN B1 CONCENTRATIONS (ng/mL) IN MILK OF LACTATING COWS DOSED TOPICALLY | | | | | | | | |
|---|---|---|---|---|---|---|---|---|
| TREATMENT A: MIGLYOL™ 840/BHT/500 ug/kg | | | | | | | | |
| ANIMAL # | DAY POST DOSE | | | | | | | |
| | 0 | 1 | 2 | 3 | 4 | 5 | 6 | 7 |
| 5950 | 0.0 | 1.5 | 5.0 | 6.4 | 9.5 | 8.7 | 8.1 | 6.3 |
| 5931 | 0.0 | 6.0 | 23.2 | 13.0 | 7.1 | 4.2 | 2.5 | 1.7 |
| 5932 | 0.0 | 3.4 | 4.8 | 3.4 | 3.1 | 2.0 | 1.6 | 3.4 |
| 5938 | 0.0 | 5.6 | 15.5 | 9.6 | 8.5 | 4.5 | 3.7 | 3.1 |
| MEAN | 0.0 | 4.1 | 12.1 | 8.1 | 7.1 | 4.9 | 4.0 | 3.6 |
| STD. | | 2.1 | 8.9 | 4.1 | 2.8 | 2.8 | 2.9 | 1.9 |
| DEV. | | | | | | | | |

| TREATMENT B: TRIACETIN/MIGLYOL™ 840 (50/50)/500 ug/kg | | | | | | | | |
|---|---|---|---|---|---|---|---|---|
| ANIMAL # | DAY POST DOSE | | | | | | | |
| | 0 | 1 | 2 | 3 | 4 | 5 | 6 | 7 |
| 5946 | 0.0 | 1.2 | 2.9 | 4.0 | 5.0 | 4.7 | 4.1 | 2.9 |
| 5949 | 0.0 | 2.7 | 13.3 | 11.4 | 8.6 | 5.3 | 3.5 | 2.8 |
| 5929 | 0.0 | 1.3 | 2.8 | 4.1 | 5.8 | 5.7 | 4.2 | 3.0 |
| 5928 | 0.0 | 4.9 | 14.6 | 9.4 | 5.4 | 3.1 | 2.0 | 1.4 |
| MEAN | 0.0 | 2.5 | 8.4 | 7.2 | 6.2 | 4.7 | 3.5 | 2.5 |
| STD. | | 1.7 | 6.4 | 3.8 | 1.6 | 1.1 | 1.0 | 0.8 |
| DEV. | | | | | | | | |

| TREATMENT C: SOFTIGEN™ 767/MIGLYOL™ 840 (70/30)/500 ug/kg | | | | | | | | |
|---|---|---|---|---|---|---|---|---|
| ANIMAL # | DAY POST DOSE | | | | | | | |
| | 0 | 1 | 2 | 3 | 4 | 5 | 6 | 7 |
| 5948 | 0.0 | 1.1 | 4.5 | 6.4 | 6.6 | 7.1 | 5.4 | 3.8 |
| 5930 | 0.0 | 1.4 | 3.8 | 3.9 | 5.8 | 9.0 | 7.6 | 4.9 |
| 5927 | 0.0 | 2.7 | 6.0 | 7.0 | 7.6 | 5.5 | 4.6 | 3.7 |
| 5934 | 0.0 | 1.9 | 4.7 | 10.6 | 15.0 | 8.3 | 4.9 | 3.2 |
| MEAN | 0.0 | 1.8 | 4.8 | 7.0 | 8.8 | 7.5 | 5.6 | 3.9 |
| STD. | | 0.7 | 0.9 | 3.9 | 4.2 | 1.5 | 1.4 | 0*.*7 |
| DEV. | | | | | | | | |

| TREATMENT D: Miglyol™/Crodamol™ CAP (90/10)-500 µg/Kg | | | | | | | | |
|---|---|---|---|---|---|---|---|---|
| ANIMAL # | DAY POST DOSE | | | | | | | |
| | 0 | 1 | 2 | 3 | 4 | 5 | 6 | 7 |
| 6384 | 0.0 | 2.1 | 4.8 | 6.8 | 7.6 | 5.7 | 5.4 | 3.8 |
| 6385 | 0.0 | 7.3 | 7.1 | 6.1 | 4.8 | 3.3 | 2.7 | 2.3 |
| 6379 | 0.0 | 10.0 | 10.6 | 7.8 | 5.4 | 2.9 | 1.9 | 1.7 |
| 6386 | 0.0 | 2.7 | 6.0 | 6.0 | 5.8 | 4.1 | 5.4 | 5.2 |
| 6377 | 0.0 | 5.2 | 9.7 | 8.7 | 9.8 | 4.8 | 2.9 | 2.5 |
| 6382 | 0.0 | 7.7 | 15.5 | 11.8 | 8.7 | 4.7 | 3.3 | 2.3 |
| MEAN | 0.0 | 5.8 | 9.0 | 7.9 | 7.0 | 4.3 | 3.6 | 3.0 |
| STD. | | 3.1 | 3.9 | 2.2 | 2.0 | 1.0 | 1.5 | 1.3 |
| DEV. | | | | | | | | |

| TREATMENT E: Miglyol™/Crodamol™ CAP (90/10)-250 µg/Kg | | | | | | | | |
|---|---|---|---|---|---|---|---|---|
| ANIMAL # | DAY POST DOSE | | | | | | | |
| | 0 | 1 | 2 | 3 | 4 | 5 | 6 | 7 |
| 6389 | 0.0 | 1.2 | 2.7 | 2.8 | 2.6 | 1.9 | 1.7 | 1.4 |
| 6381 | 0.0 | 1.0 | 1.6 | 1.8 | 2.8 | 2.5 | 2.6 | 2.0 |
| 6380 | 0.0 | 2.8 | 5.5 | 4.4 | 3.5 | 2.0 | 1.5 | 0.0 |
| 6378 | 0.0 | 2.4 | 4.8 | 4.0 | 2.9 | 1.6 | 1.1 | 0.0 |
| 6376 | 0.0 | 1.8 | 4.2 | 4.3 | 4.3 | 3.1 | 2.4 | 1.9 |
| 6388 | 0.0 | 0.0 | 1.3 | 1.7 | 2.8 | 2.3 | 2.0 | 1.9 |
| MEAN . | 0.0 | 1.5 | 3.4 | 3.2 | 3.2 | 2.2 | 1.9 | 1.2 |
| STD. | | 1.0 | 1.7 | 1.2 | 0.6 | 0.5 | 0.6 | 1.0 |
| DEV. | | | | | | | | |

| TREATMENT F: TRIACETIN/MIGLYOL™ 840 (50/50)/500 ug/kg | | | | | | | | |
|---|---|---|---|---|---|---|---|---|
| ANIMAL # | DAY POST DOSE | | | | | | | |
| | 0 | 1 | 2 | 3 | 4 | 5 | 6 | 7 |
| 5977 | 0.0 | 1.4 | 6.8 | 6.9 | 4.3 | 3.4 | 3.0 | 2.2 |
| 5976 | 0.0 | 2.1 | 10.8 | 13.8 | 5.7 | 5.6 | 2.9 | 1.8 |
| MEAN | 0.0 | 1.8 | 8.8 | 10.4 | 5.0 | 4.5 | 3.0 | 2.0 |
| STD. | | 0.5 | 2.8 | 4.9 | 1.0 | 1.6 | 0.0 | 0.3 |
| DEV. | | | | | | | | |

| TREATMENT :G IPA/SOFTIGEN™ 767 (40/60)/500 ug/kg | | | | | | | | |
|---|---|---|---|---|---|---|---|---|
| ANIMAL # | DAY POST DOSE | | | | | | | |
| | 0 | 1 | 2 | 3 | 4 | 5 | 6 | 7 |
| 5984 | 0.0 | 0.0 | 0.0 | 0.6 | 0.6 | 0.7 | 1.0 | 1.6 |
| 5980 | 0.0 | 0.5 | 0.8 | 1.6 | 1.3 | 2.9 | 2.3 | 2.8 |
| 5987 | 0.0 | 0.0 | 0.5 | 0.9 | 3.1 | 6.3 | 4.6 | 5.0 |
| 5982 | 0.0 | 0.0 | 2.0 | 3.8 | 3.8 | 3.5 | 2.2 | 1.6 |
| MEAN | 0.0 | 0.0 | 0.8 | 1.7 | 2.2 | 3.4 | 2.5 | 2.8 |
| STD. | | 0.0 | 0.9 | 1.5 | 1.5 | 2.3 | 1.5 | 1.6 |
| DEV. | | | | | | | | |
| NOTE: Samples with 4"-acetylamino-4"-deoxyavermectin B1 concentrations equal to or less than 0.4 ng/ml are reported as 0 ng/ml: | | | | | | | | |

### E1 EXAMPLE III

Efficacy trials with Chorioptes and key endoparasites were conducted to evaluate some of the above formulations. For each trial evaluating Chorioptes, four cattle were infested with *Chorioptes bovis* on Day -1 and treatment was given on Day 0. Respecting the trials evaluating endoparasites, the animals were challenged with Oesophagostamum, Trichuris and Dictyocaulus 17, 7, and 7 days before treatment with the formulation. The results are below.

| CHORIOPTES MITE COUNTS | | | | | | |
|---|---|---|---|---|---|---|
| An. No. | Day -1 | Day 7 | Day 14 | Day 21 | Day 27 | Day 35 |
| Trt. 1 - Untreated Control | | | | | | |
| H215 | 32 | 3 | 0 | 10 | 0 | 0 |
| H208 | 6 | 19 | 15 | 3 | 4 | 17 |
| H229 | 708 | 5024 | 2546 | 601^{a} | 11477^{b} | 6835 |
| H233 | 511 | 875 | 1430 | 889^{a} | 1432^{b} | 1339 |

| Trt. 2 - 4"-aa-4" deoxy in Miglyol™ 840/Crodamol™ CAP/BHT at 500 mcg/kg | | | | | | |
|---|---|---|---|---|---|---|
| H224 | 22 | 1 | 79 | 2 | 0 | 0 |
| H223 | 17 | 0 | 0 | 0 | 0 | 0 |
| H234 | 2018 | 1007 | 895 | 0^{a} | 0^{b} | 0 |
| H230 | 378 | 265 | 2 TM | 5^{a} | 0^{b} | 150 |

| Trt. 3 - 4"-aa-4" deoxy in Miglyol™ 840/BHT at 500 mcg/kg | | | | | | |
|---|---|---|---|---|---|---|
| H226 | 182 | 3 | 0 | 1 | 0 | 0 |
| H218 | 3 | 0 | 0 | 0 | 0 | 0 |
| H228 | 1644 | 659 | 16 | 0^{a} | 0^{b} | 0 |
| H231 | 233 | 358 | 603 | 133^{a} | 89^{b} | 5 |

| | | | | | | |
|---|---|---|---|---|---|---|
| ^{a} Day 20 | | | | | | |
| ^{b} Day 28 4"-aa-4" deoxy = 4"-acetylamino-4"-deoxy avermectin B1 | | | | | | |

| 4"-aa-4" deoxy Nematode Counts Total Counts based on 10% aliquots (*Dictyocaulus* counts are total counts) | | | | |
|---|---|---|---|---|
| Animal Number | *Oesophagost* spp. Adult | *Oesophagost* spp. L4 | *Trichuris* spp. Adult | *Dictyocaulus* spp. |
| Trt. 1 - Untreated Control | | | | |
| 2477 | 0 | 0 | 20 | 3 |
| 2374 | 50 | 0 | 0 | 0 |
| 2259 | 0 | 0 | 50 | 17 |
| 41 | 240 | 0 | 80 | 14 |

| Trt. 2 - 4"-aa-4" deoxy in Miglyol™ 840 Crodamol™ CAP/BHT (0.5%/q.s./10%/0.01% at 500 mcg/kg | | | | |
|---|---|---|---|---|
| 2456 | 0 | 0 | 0 | 0 |
| 2478 | 0 | 0 | 0 | 0 |
| 5 | 0 | 0 | 0 | 0 |
| 2254 | 0 | 0 | 0 | 0 |

| Trt. 3 - 4"-aa-4" deoxy in Miglyol 840 BHT (0.5%/q.s./0.01 %) at 500 mcg/kg | | | | |
|---|---|---|---|---|
| 2510 | 0 | 0 | 0 | 0 |
| 2358 | 0 | 0 | 0 | 0 |
| 40 | 0 | 0 | 0 | 0 |
| 2258 | 0 | 0 | 0 | 0 |

| Trt. 4 - 4"-aa-4" deoxy in Miglyol™ 840/Lauroglycol/BHT (0.5%/q.s./10%/0.01 %) at 500 mcg/kg | | | | |
|---|---|---|---|---|
| 2528 | 0 | 0 | 0 | 0 |
| 2443 | 0 | 0 | 0 | 0 |
| 44 | 0 | 0 | 0 | 0 |
| 2298 | 0 | 0 | 0 | 0 |

Another preferred embodiment (E2) of the instant formulation consists of a topical pour-on formulation of a solvent mixture of water and/or solvents with relative high vapor pressure such as ethanol, methanol, isopropanol, acetone, and the like, most preferrably ethanol, a polymeric material such as polyvinyl pyrrolidone, polyvinyl alcohol, cellulose derivatives such as methyl cellulose, ethyl cellulose, carboxy methyl cellulose, and hydroxyethyl cellulose, and the like, most preferrably polyvinyl pyrrolidone (MW from 20,000 to 65,000, preferrably 45,000), skin or hair subtantantive protein derivatives such as hydrolyzed wheat protein, hydrolyzed animal protein, gelatin derivatives, collagen derivatives, and the like, hydroalcoholic soluble copolymers such as acrylates/t-octylpropenamide copolymer and the like, and cationic quaternary amine salts and the like, which has been found to extended the efficacy of the formulation for up to a full four weeks. The polymeric material helps to keep the drug at the skin level longer by remaining on the skin surface after the solvents have evaporated following application. The remaining avermectin and polymer does not change the appearance of the animal's hair coat and the avermectin is released by diffusion and/or erosion of the polymer.

The preferred avermectin compounds of E2 have the following structural formula: 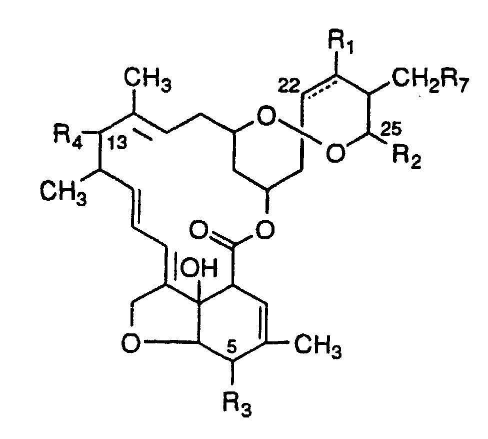 wherein R₁, R₂, and R₃ are as described above, and R₄ is hydrogen, hydroxy, or polyalkoxy, and the broken line indicates a single or double bond at the 22,23-position, provided that R2 is hydroxy only when the broken line indicates a single bond.

Examples of preferred compounds of the instant invention are:
4"-keto avermectin B1;
4"-keto avermectin B2;
4"-keto-22,23-dihydro avermectin B1;
4"-keto-22,23-dihydro avermectin B2;
4"-deoxy-4"-amino avermectin B1;
4"-deoxy-4"-amino avermectin B2;
4"-deoxy-4"-amino-22,23-dihydro avermectin B1;
4"-deoxy-4"-acetylamino avermectin B1;
4"-deoxy-4"-acetylamino avermectin B2;
4"-deoxy-4"-acetylamino-22,23-dihydro avermectin B1;
4"-deoxy-4"-acetylamino-22,23-dihydro avermectin B2;
4"-deoxy-4"-dimethylamino avermectin B1;
4"-deoxy-4"-dimethylamino avermectin B2;
4"-deoxy-4"-dimethylamino-22,23-dihydro avermectin B1;
4"-deoxy-4"-dimethylamino-22,23-dihydro avermectin B2;
4"-deoxy-4"-p-chloro benzenesulfonylamino-22,23-dihydro avermectin B1;
4"-deoxy-4"-p-chloro benzenesulfonylamino-22,23-dihydro-13-O-[(2-methoxyethoxy)methyl] avermectin B1 aglycone (hereinafter referred to as 13-O-MEM AVM);
4"-deoxy-4"-(2-methylbenzenesulfonylamino)-avermectin B1;
4"-deoxy-4"-(2-methylbenzenesulfonylamino)-avermectin B2
13-epi-O-(methoxymethyl)-22,23-dihydro avermectin B1 aglycone (hereinafter referred to as 13-O-MOM AVM).
The most preferred compound is 22,23-dihydro-13-O-[(2-methoxyethoxy)methyl] avermectin B1 aglycone (hereinafter referred to as 13-O-MEM AVM).

In the topical forms of the avermectin formulation it has not been possible to provide a formulation which provides superior extended efficacy against ectoparasites, especially fleas and ticks. Additionally, currently available topical formulations do not provide adequate efficacy against endoparasites, especially heartworms and nematodes.

The E2 embodiment of the instant formulation gives the advantages of a pour-on topical formulation which provides the animal with extended effective treatment and protection against endoparasites and ectoparasites, especially fleas, ticks, mange mites, hookworms, ascarids, and heartworms. Additional advantages of this invention are that the formulation is not readily dislodgeable by petting the animals, it has good spreadability and cold temperature usage.

The E2 embodiment of the instant formulation can contain the avermectin compound, alcohol, water and the polymer as the only ingredients. The formulations will benerally be prepared to administered the avermectin from 0.005 by weight to 30% of the total composition, preferrably from 0.1 to 10% by weight and most preferrably 5% by weight of the active ingredient. At a preferred dose of 0.5 to 50 mg/kg the formulation is applied at a dose volume of 0.05 to 4.0 ml/kg body weight. The polymer is present in the compositions of the present invention in amounts ranging from 0% to 20% w/v and preferrably from 0.5 to 10% w/v by weight of the total composition and up to 95% by volume of alcohol, q.s. to 100% with water.

The preferred E2 embodiment contains in addition to the polymer, alcohol, water and avermectin compound, additional ingredients such as antioxidants and the glycol, glycerides, glycol ethers, and the derivatives thereof mentioned above. The anti-oxidants are generally added to the formulation at rates of from 0.005 to 1.0% (w/v) and can be propyl gallate, BHA (butylated hydroxy anisole), BHT (butylated hydroxy toluene), monothioglycerol and the like, preferrably BHT.

The E2 formulation is prepared by dissolving the avermectin compound in the intended volume of alcohol. The anti-oxidant and one of the polymeric materials listed above are then dissolved in the alcohol/avermectin mixture. The volume is then adjusted to 100% by the addition of the final volume of water, with the solution being mixed until it becomes homogeneous. Alternatively, either the BHT or the polymer, or both can be added prior to the addition of the avermectin compound.

The following example is provided in order that the E2 emobodiment of the invention might be more fully understood.

### EXAMPLE OF E2 OF THE INVENTION

The E2 formulations of this invention which are employed depend upon the particular avermectin compound and treatment. To test the effective killing power of the E2 formulations against fleas and ticks, the following compositions were prepared:

| Composition VIII | |
|---|---|
| 13-O-MEM AVM | 0.3 % w/v |
| polyvinyl pyrrolidone | 5.0 % w/v |
| Cremophor ™ RH-40 | 1.0 % w/v |
| Anhyd. (Denatured) Ethanol | 40.0 % v/v |
| Softigen™ 767 | 20.0 % v/v |
| Water (q.s.) | 100.0 % v/v |

| Composition IX | |
|---|---|
| 13-O-MEM AVM | 0.3 % w/v |
| polyvinyl pyrrolidone | 5.0 % w/v |
| Anhydrous Ethanol | 75.0 % v/v |
| Water (q.s.) | 100.0 % v/v |
| BHT | 0.01% w/v |

| Composition X | |
|---|---|
| 13-O-MOM AVM | 5.0 % w/v |
| polyvinyl pyrrolidone | 5.0 % w/v |
| Anhydrous Ethanol | 90.0 % v/v |
| Water (q.s.) | 100.0 % v/v |
| BHT | 0.01% w/v |

| Composition XI | |
|---|---|
| 13-O-MEM AVM | 0.6 % w/v |
| polyvinyl pyrrolidone | 5.0 % w/v |
| Anhydrous Ethanol | 75.0 % v/v |
| Water (q.s.) | 100.0 % v/v |
| Vitamin E | 0.02% v/v |

| Composition XII | |
|---|---|
| 13-O-MEM AVM | 0.6 % w/v |
| hydrolyzed wheat protein | 3.0 % w/v |
| Anhydrous Ethanol | 90.0 % v/v |
| Water (q.s.) | 100.0 % v/v |
| Vitamin E | 0.02% v/v |

| Composition XIII | |
|---|---|
| 13-O-MEM AVM | 0.6 % w/v |
| Ethocel | 2.0 % w/v |
| Anhydrous Ethanol | 90.0 % v/v |
| Water (q.s.) | 100.0 % v/v |
| Vitamin E | 0.02% v/v |

| Composition XIV | |
|---|---|
| 13-O-MEM AVM | 0.6 % w/v |
| polyvinyl pyrrolidine | 5.0 % w/v |
| Anhydrous Ethanol | 80.0 % v/v |
| Water (q.s.) | 100.0 % v/v |
| Vitamin E | 0.02% v/v |
| Miglyol™ | 0.5 % v/v |

| Composition XV | |
|---|---|
| 13-O-MEM AVM | 0.6 % w/v |
| acrylates/t-octylpropenamide copoly | 1.0 % w/v |
| polyvinyl pyrrolidone | 2.0 % w/v |
| Anhydrous Ethanol | 80.0 % v/v |
| Water (q.s.) | 100.0 % v/v |
| Vitamin E | 0.02% v/v |

| Composition XVI | |
|---|---|
| 13-O-MEM AVM | 5.0 % w/v |
| polyvinyl pyrrolidone (m.wt. 45,000) | 5.0 % w/v |
| anhydrous ethanol | 90.0 % v/v |
| water (q.s.) | 100.0 % v/v |
| BHT | 0.01 % w/v |

Softigen™ 767 is a tradename for PEG-6 caprylic/caprate glyceride, Cremophor RH-40 is a tradename for a mixture of glycerol polyethylene and glycol oxysteasrate, and Ethocel is a tradename for ethyl cellulose.

Composition X above was topically applied in multiple locations, typically 2 to 6 points spaced equidistant between the back of the neck and the head of the tail of a flea infested dog. Counts were made by combing the hair, removing and counting the live parasites on the dog at a specified time. The observed flea kills varying the amount of 13-O-MEM AVM, is given in Table No. 1 below, where 60 dogs were allocated to four treatment groups. The dogs were infested with 100 unfed, adult fleas at times indicated by the down arrow ( ), which is equivalent to three days before a flea count is conducted. Treatment was applied on day zero. Table No. 2 summarizes the results of a similar test evaluating the efficacy of the composition, containing 13-O-MEM AVE (termed 2-MEM) in various vehicles, in the treatment of ticks.

The instant formulations can be topically administered to warm blooded animals to provide long acting treatment and protection against endoparasites and ectoparasites either locally at the site of infestation or at multiple points, typically 2 to 6 points (multiple-point-application) along the back of domesticated animals and household pets such as cattle, sheep, cats, dogs and the like.

## Claims

1. A topical pour-on formulation against ectoparasites and endoparasites, which provides a zero-milk withdrawal time in dairy animals, which comprises from 0.005 to 10% w/v of an avermectin compound having the formula: 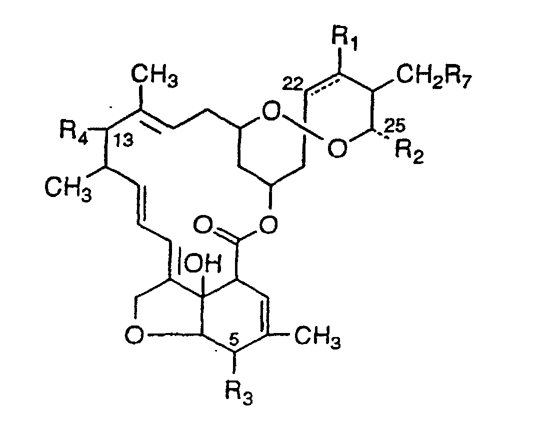 where the broken line indicates a single or a double bond at the 22,23-positions;
R₁ is hydrogen or hydroxy provided that R₁ is present only when the broken line indicates a single bond;
R₂ is alkyl of from 1 to 6 carbon atoms or alkenyl of from 3 to 6 carbon atoms or cycloalkyl of from 3 to 6 carbon atoms;
R₃ is hydroxy, methoxy or =NOR₅ where R₅ is hydrogen or lower alkyl;
R₇ is hydrogen, hydroxy or loweralkyl (C₁-C₅); and
R₄ is hydrogen, hydroxy, C(1-6) polyalkoxy or 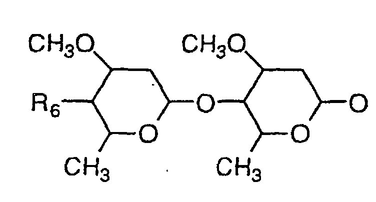 where R₆ is hydroxy, amino, mono- or di-C₁-C₆ alkylamino or C₁-C₆ alkanoylamino; from 0.005 to 1.0% w/v of an anti-oxidant and from 40% to 100% q.s. v/v of a glyceride carrier selected from the group consisting of propylene dicaprylate/dicaprate, or caprylic/capric triglyceride.

2. The formulation of Claim 1 wherein R₄ is 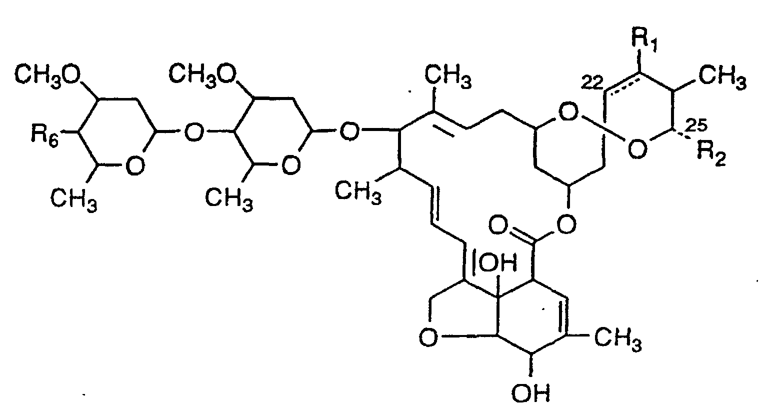 wherein R₁, R₂, and R₆ are stated as in Claim 1.

3. The formulation of Claim 1 which contains from 0.01 to 5% w/v of the avermectin compound.

4. The formulation of any one of Claims 1 to 3 wherein the antioxidant is selected from the group consisting of n-propyl fallate, BHA, BHT, or monothioglycerol.

5. The formulation of Claim 4 wherein the antioxidant is BHT.

6. The formulation of Claim 1 which optionally contains a solvent belonging to the group consisting of Crodamol Cap®, glycerol formal, Tween 80, or propylene glycol at up to 60% v/v.

7. The formulation of Claim 1 consisting of 100% q.s., v/v propylene dicaprylate/dicaprate or caprylate/caprate glyceride, from 0.005 to 0.05% w/v BHT and from 0.01 to 5% w/v of 4"-acetylamino-4"-deoxyavermectin B1.

8. The formulation of Claim 7 consisting 0.5% w/v of 4"-acetylamino-4"-deoxyavennectin B1, and 0.01% w/v BHT.

9. A process for the preparation of the formulation of Claim 1 which comprises dissolving the avermectin compound of Claim 1 in 50% q.s., v/v of the total volume of the glyceride carrier, adding the anti-oxidant thereto and adding as a final volume, the remainder of the carrier.

10. A topical formulation for direct application to the skin of an animal, effective for treatment and prevention of ectoparasites and endoparasitic infestations for four weeks, consisting of from 0.01 to 20% w/v of a polymeric material, from 1 to from 95% v/v of ethanol, 100% v/v obtained with addition of water, and from 0.05 to from 10% w/v of an avermectin compound having the formula: 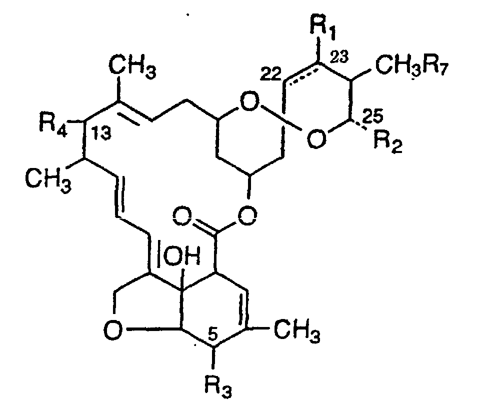 where the broken line indicates a single or a double bond at the 22,23-positions;
R₁ is hydrogen or hydroxy, provided that R₁ is present only when the broken line indicates a single bond;
R₂ is alkyl of from 1 to 6 carbon atoms or alkenyl of from 3 to 6 carbon atoms or cycloalkyl of from 3 to 6 carbon atoms;
R₃ is hydroxy, methoxy, or =NOR₅; where R₅ is hydrogen or lower alkyl;
R₇ is hydrogen, hydroxy or loweralkyl (C₁-C₅), and
R₄ is hydrogen, hydroxy, poly C(1-6) alkoxy, or 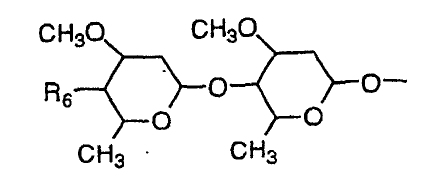 where R₆ is hydroxy, amino, mono- or di-C₁-C₆ alkylamino or C₁-C₆ alkanoylamino.

11. The formulation of Claim 10 wherein R₄ is hydrogen, hydroxy or polyalkoxy.

12. The formulation of Claim 10 which contains from 0.1 to 5.0% w/v of the avermectin compound and 5.0 to 10% w/v of the polymeric material and wherein R₄ of the avermectin compound is CH3OCH2CH2OCH2O.

13. The formulation of Claim 10 wherein the polymeric material is selected from the group consisting of polyvinyl pyrrolidone, polyvinyl alchohol, methyl cellulose, ethyl cellulose, carboxy methyl cellulose, hydroxyethyl cellulose, hydrolyzed wheat protein, hydrolyzed animal protein, gelatin derivatives, collagen derivatives, acrylates/t-octylpropenamide copolymer and cationic quaternary amine salts.

14. The formulation of Claim 13 wherein the polymeric material is polyvinyl pyrrolidone.

15. The formulation of Claim 14 wherein the polyvinyl pyrrolidone has a molecular weight of 20,000 to 65,000.

16. The formulation of Claim 15 wherein the polyvinyl pyrrolidone has a molecular weight of 45,000.

17. The formulation of Claim 10 which optionally contains an anti-oxidant selected from the group consisting of n-propyl gallate, BHA, BHT and monothioglycerol from 0.005 to 1.0% w/v.

18. The formulation of Claim 10 which optionally contains a glycol, glyceride or glycol ether.

19. A topical formulation for direct application to the skin of an animal for effective treatment of parasitic infestations consisting of 5.0% w/v of polyvinyl pyrrolidone, molecular weight 45,000, 5.0% w/v of 22,23-dihydro-13-O-[(2-methoxyethoxy)methyl] avermectin B1 aglycone, 90% v/v ethanol q.s. to 100% with water and 0.01% w/v BHT.

20. A process for the preparation of the formulation of Claim 10 which comprises dissolving from 0.005 to from 10% w/v of the avermectin compound of Claim 1 in from 1 to from 95% v/v of ethanol to form a clear solution, adding and dissolving from about 0.005 to 1.0% w/v of the anti-oxidant and from 0.01 to from 20% w/v of the polymeric material in the solution, adding up to 50% v/v of the additive, adjusting the volume to 100% by addition of water and mixing until the solution is homogeneous.

## Patentansprüche

1. Eine topische Pour-On-Formulierung gegen Ektoparasiten und Endoparasiten ohne Wartezeit für Milch bei Milchvieh, die 0,005 bis 10% Gew./Vol. einer Avermectinverbindung mit der Formel: 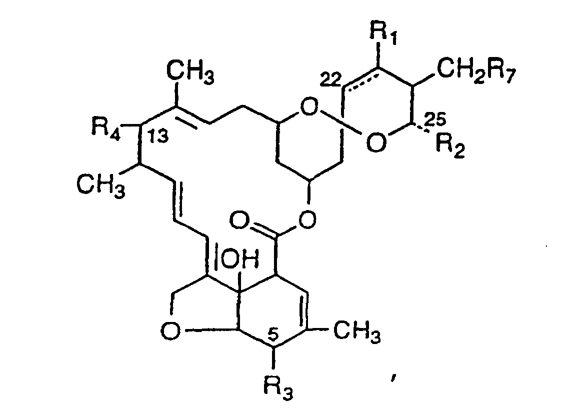 wobei die gestrichelte Linie eine Einfach- oder eine Doppelbindung in der 22,23-Stellung bedeutet,
R₁ Wasserstoff oder Hydroxy ist, mit der Maßgabe, daß R₁ nur vorhanden ist, wenn die gestrichelte Linie eine Einfachbindung bedeutet,
R₂ Alkyl mit 1 bis 6 Kohlenstoffatomen oder Alkenyl mit 3 bis 6 Kohlenstoffatomen oder Cycloalkyl mit 3 bis 6 Kohlenstoffatomen ist,
R₃ Hydroxy, Methoxy oder =NOR₅ ist, wobei R₅ Wasserstoff oder Niedrigalkyl ist,
R₇ Wasserstoff, Hydroxy oder Niedrigalkyl (C₁-C₅) ist und
R₄ Wasserstoff, Hydroxy, C(1-6)-Polyalkoxy oder 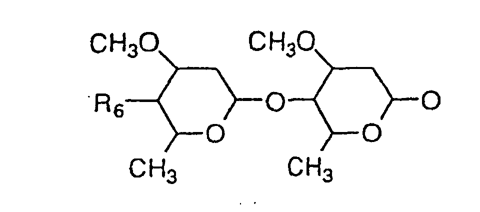 ist, wobei R₆ Hydroxy, Amino, Mono- oder Di-C₁-C₆-alkylamino oder C₁-C₆-Alkanoylamino ist, 0,005 bis 1,0% Gew./Vol. eines Antioxidationsmittels und 40% bis 100% q.s. Vol./Vol. eines Glyceridträgers, ausgewählt aus der Gruppe, bestehend aus Propylendicaprylat/Dicaprat oder Capryl/Caprintriglycerid, enthält.

2. Die Formulierung nach Anspruch 1, wobei R₄ 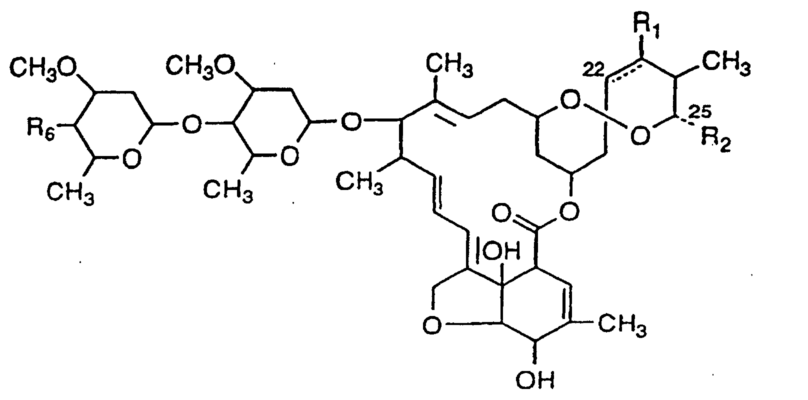 ist, wobei R₁, R₂ und R₆ wie in Anspruch 1 angegeben sind.

3. Die Formulierung nach Anspruch 1, die 0,01 bis 5% Gew./Vol. der Avermectinverbindung enthält.

4. Die Formulierung nach irgendeinem der Ansprüche 1 bis 3, wobei das Antioxidationsmittel ausgewählt ist aus der Gruppe, bestehend aus n-Propylfallat, BHA, BHT oder Monothioglycerin.

5. Die Formulierung nach Anspruch 4, wobei das Antioxidationsmittel BHT ist.

6. Die Formulierung nach Anspruch 1, die gegebenenfalls bis zu 60% Vol./Vol. eines Lösungsmittels enthält, das zur Gruppe gehört, die aus Crodamol Cap®, Glycerinformal, Tween 80 oder Propylenglycol besteht.

7. Die Formulierung nach Anspruch 1, bestehend aus 100% q.s. Vol./Vol. Propylendicaprylat/Dicaprat oder Caprylat/Capratglycerid, 0,005 bis 0,05% Gew./Vol. BHT und 0,01 bis 5% Gew./Vol. 4"-Acetylamino-4"-desoxyavermectin B1.

8. Die Formulierung nach Anspruch 7, die aus 0,5% Gew./Vol. 4"-Acetylamino-4"-desoxyavermectin B1 und 0,01% Gew./Vol. BHT besteht.

9. Ein Verfahren zur Herstellung der Formulierung nach Anspruch 1, welches das Auflösen der Avermectinverbindung nach Anspruch 1 in 50% q.s. Vol/Vol. des Gesamtvolumens des Glyceridträgers, die Zugabe des Antioxidationsmittels dazu und die Zugabe des Restes des Trägers als ein letztes Volumen umfaßt.

10. Eine topische Formulierung zum direkten Auftrag auf die Haut eines Tieres, die zur Behandlung und Prävention von Ektoparasiten- und Endoparasitenbefall für vier Wochen wirksam ist, bestehend aus 0,01 bis 20% Gew./Vol. eines polymeren Materials, 1 bis 95% Vol./Vol. Ethanol, 100% Vol./Vol., die durch die Zugabe von Wasser erzielt werden, und 0,05 bis 10% Gew./Vol. einer Avermectinverbindung mit der Formel: 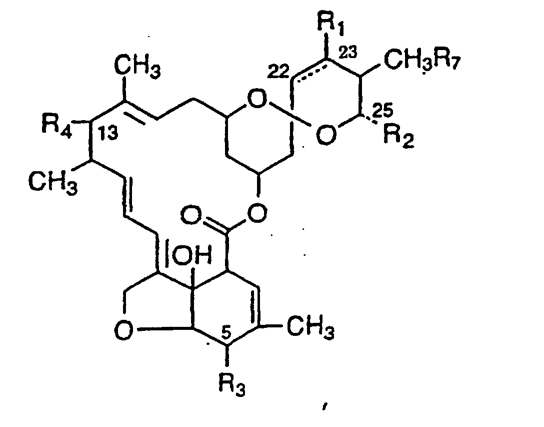 wobei die gestrichelte Linie eine Einfach- oder eine Doppelbindung in der 22,23-Stellung bedeutet,
R₁ Wasserstoff oder Hydroxy ist, mit der Maßgabe, daß R₁ nur vorhanden ist, wenn die gestrichelte Linie eine Einfachbindung bedeutet,
R₂ Alkyl mit 1 bis 6 Kohlenstoffatomen oder Alkenyl mit 3 bis 6 Kohlenstoffatomen oder Cycloalkyl mit 3 bis 6 Kohlenstoffatomen ist,
R₃ Hydroxy, Methoxy oder =NOR₅ ist, wobei R₅ Wasserstoff oder Niedrigalkyl ist,
R₇ Wasserstoff, Hydroxy oder Niedrigalkyl (C₁-C₅) ist und
R₄ Wasserstoff, Hydroxy, Poly-C(1-6)-alkoxy oder 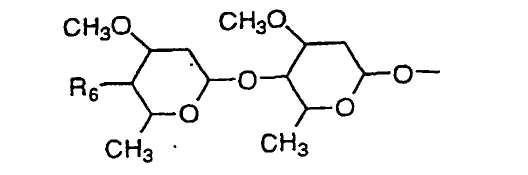 ist, wobei R₆ Hydroxy, Amino, Mono- oder Di-C₁-C₆-alkylamino oder C₁-C₆-Alkanoylamino ist.

11. Die Formulierung nach Anspruch 10, wobei R₄ Wasserstoff, Hydroxy oder Polyalkoxy ist.

12. Die Formulierung nach Anspruch 10, die 0,1 bis 5,0% Gew./Vol. der Avermectinverbindung und 5,0 bis 10% Gew./Vol. des polymeren Materials enthält, und wobei R₄ der Avermectinverbindung CH₃OCH₂CH₂OCH₂O ist.

13. Die Formulierung nach Anspruch 10, wobei das polymere Material ausgewählt ist aus der Gruppe, bestehend aus Polyvinylpyrrolidon, Polyvinylalkohol, Methylcellulose, Ethylcellulose, Carboxymethylcellulose, Hydroxyethylcellulose, hydrolysiertem Weizeneiweiß, hydrolysiertem Tiereiweiß, Gelatinederivaten, Kollagenderivaten, Acrylate/t-Octylpropenamid-Copolymer und kationischen quaternären Aminsalzen.

14. Die Formulierung nach Anspruch 13, wobei das polymere Material Polyvinylpyrrolidon ist.

15. Die Formulierung nach Anspruch 14, wobei das Polyvinylpyrrolidon ein Molekulargewicht von 20000 bis 65000 hat.

16. Die Formulierung nach Anspruch 15, wobei das Polyvinylpyrrolidon ein Molekulargewicht von 45000 hat.

17. Die Formulierung nach Anspruch 10, die gegebenenfalls 0,005 bis 1,0% Gew./Vol. eines Antioxidationsmittels, ausgewählt aus der Gruppe, bestehend aus n-Propylgallat, BHA, BHT und Monothioglycerin, enthält.

18. Die Formulierung nach Anspruch 10, die gegebenenfalls ein Glycol, Glycerid oder einen Glycolether enthält.

19. Eine topische Formulierung zum direkten Auftrag auf die Haut eines Tieres, die zur Behandlung von Parasitenbefall wirksam ist, bestehend aus 5,0% Gew./Vol. Polyvinylpyrrolidon, Molekulargewicht 45000, 5,0% Gew./Vol. 22,23-Dihydro-13-O-[(2-Methoxyethoxy)-methyl]avermectin-B1-Aglykon, 90% Vol./Vol. Ethanol, q.s. auf 100% mit Wasser und 0,01% Gew./Vol. BHT.

20. Ein Verfahren zur Herstellung der Formulierung nach Anspruch 10, welches das Auflösen von 0,005 bis 10% Gew./Vol. der Avermectinverbindung nach Anspruch 1 in 1 bis 95% Vol./Vol. Ethanol, um eine klare Lösung zu bilden, die Zugabe und das Auflösen von etwa 0,005 bis 1,0% Gew./Vol. des Antioxidationsmittels und 0,01 bis 20% Gew./Vol. des polymeren Materials in der Lösung, die Zugabe von bis zu 50% Vol./Vol. des Additivs, das Einstellen des Volumens auf 100% durch Zugabe von Wasser und das Vermischen, bis die Lösung homogen ist, umfaßt.

## Revendications

1. Formulation topique à verser contre les ectoparasites et les endoparasites, qui fournit un délai de sevrage de lait nul chez les animaux laitiers, qui comprend de 0,005 à 10% en poids/volume d'un composé d'avermectine ayant la formule : 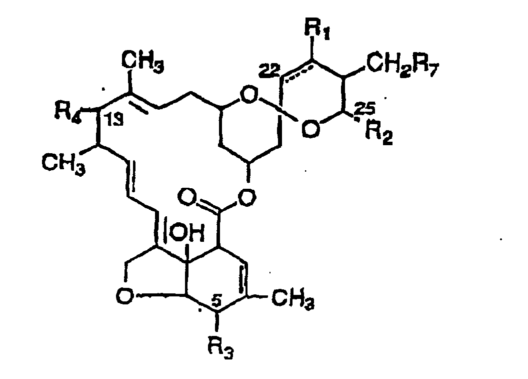 où la ligne brisée indique une liaison simple ou une liaison double aux positions 22,23 ;
R₁ est un atome d'hydrogène ou un groupe hydroxy à condition que R₁ soit présent uniquement quand la ligne brisée indique une liaison simple ;
R₂ est un groupe alkyle de 1 à 6 atomes de carbone ou un groupe alcényle de 3 à 6 atomes de carbone ou un groupe cycloalkyle de 3 à 6 atomes de carbone ;
R₃ est un groupe hydroxy, méthoxy, ou =NOR₅ où R₅ est un atome d'hydrogène ou un groupe alkyle inférieur ;
R₇ est un atome d'hydrogène, un groupe hydroxy ou un groupe alkyle inférieur en (C₁-C₅) ; et
R₄ est un atome d'hydrogène, un groupe hydroxy, un groupe polyalcoxy en C(1-6) ou 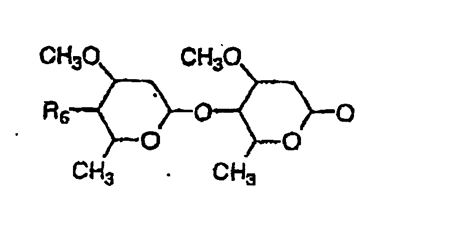 où R₆ est un groupe hydroxy, amino, mono- ou di-alkylamino en C₁ à C₆ ou alkanoylamino en C₁ à C₆ ; de 0,005 à 1,0% en poids/volume d'un antioxydant et de 40% à 100% quantité suffisante, en volume/volume d'un véhicule glycéride choisi parmi le groupe consistant en le dicaprylate/dicaprate de propylène, ou le triglycéride caprylique/caprique.

2. Formulation selon la revendication 1 dans laquelle R₄ est 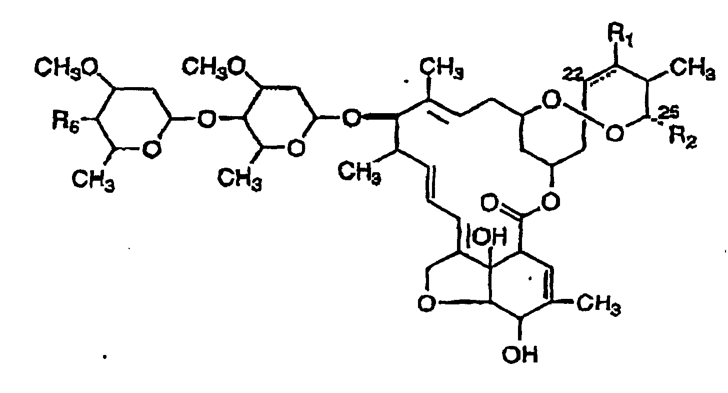 dans laquelle R₁, R₂, et R₆ sont tels que définis dans la revendication 1.

3. Formulation selon la revendication 1 qui contient de 0,01 à 5% en poids/volume du composé d'avermectine.

4. Formulation selon l'une quelconque des revendications 1 à 3 dans laquelle l'antioxydant est choisi parmi le groupe comprenant le fallate de n-propyle, le BHA, le BHT, ou le monothioglycérol.

5. Formulation selon la revendication 4 dans laquelle l'antioxydant est le BHT.

6. Formulation selon la revendication 1 qui contient facultativement un solvant appartenant au groupe comprenant le Crodamol Cap®, le glycérol formal, le Tween 80, ou le propylène glycol jusqu'à 60% en volume/volume.

7. Formulation selon la revendication 1 consistant en 100% quantité suffisante, en volume/volume de dicaprylate/dicaprate de propylène ou de glycéride caprylate/caprate, de 0,005 à 0,05% en poids/volume de BHT et de 0,01 à 5% en poids/volume de 4"-acétylamino-4"-déoxyavermectine B1.

8. Formulation selon la revendication 7 consistant en 0,5% en poids/volume de 4"-acétylamino-4"-déoxyavermectine B1, et 0,01% en poids/volume de BHT.

9. Procédé de préparation de la formulation selon la revendication 1 qui comprend les étapes consistant à dissoudre le composé d'avermectine selon la revendication 1 dans 50% quantité suffisante, en volume/volume du volume total du véhicule triglycéride, à y ajouter l'antioxydant et à ajouter comme un volume final, le reste du véhicule.

10. Formulation topique pour l'application directe sur la peau d'un animal, efficace pour le traitement et la prévention des infestations d'ectoparasites et d'endoparasites pendant quatre semaines, comprenant de 0,01 à 20% en poids/volume d'un matériau polymérique, de 1 à 95% en volume/volûme d'éthanol, 100% en volume/volume obtenu avec l'addition d'eau, et de 0,05 à 10% en poids/volume d'un composé d'avermectine ayant la formule : 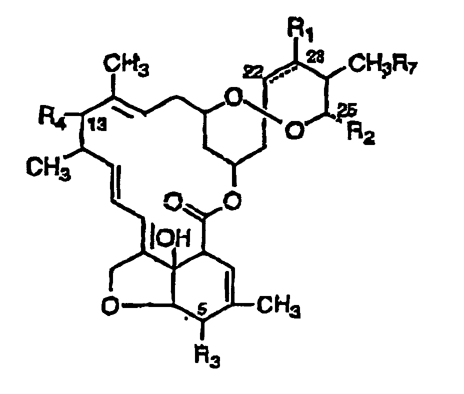 où la ligne brisée indique une liaison simple ou double aux positions 22,23 ;
R₁ est un atome d'hydrogène ou un groupe hydroxy à condition que R₁ soit présent uniquement quand la ligne brisée indique une liaison simple ;
R₂ est un groupe alkyle de 1 à 6 atomes de carbone ou un groupe alcényle de 3 à 6 atomes de carbone ou un groupe cycloalkyle de 3 à 6 atomes de carbone ;
R₃ est un groupe hydroxy, méthoxy, ou =NOR₅ où R₅ est un atome d'hydrogène ou un groupe alkyle inférieur ;
R₇ est un atome d'hydrogène, un groupe hydroxy ou un groupe alkyle inférieur en (C₁-C₅) ; et
R₄ est un atome d'hydrogène, un groupe hydroxy, un groupe polyalcoxy en C(1-6) ou 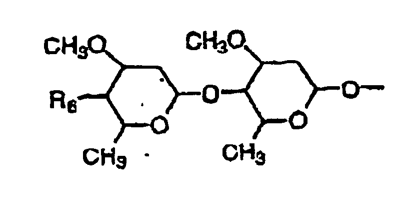 où R₆ est un groupe hydroxy, amino, mono- ou di-alkylamino en C₁ à C₆ ou alkanoylamino en C₁ à C₆.

11. Formulation selon la revendication 10 dans laquelle R₄ est un atome d'hydrogène, un groupe hydroxy ou polyalcoxy.

12. Formulation selon la revendication 10 qui contient de 0,1 à 5% en poids/volume du composé d'avermectine et de 5,0 à 10% en poids du matériau polymérique et dans laquelle R₄ du composé d'avermectine est CH₃OCH₂CH₂OCH₂O.

13. Formulation selon la revendication 10 dans laquelle le matériau polymérique est chois parmi le groupe consistant en la polyvinyle pyrrolidone, l'alcool polyvinylique, la méthyl cellulose, l'éthyl cellulose, la carboxy méthyl cellulose, l'hydroxyéthyle cellulose, de la protéine de blé hydrolysée, de la protéine animale hydrolysée, les dérivés de gélatine, les dérivés de collagène, les copolymères d'acrylates/t-octylpropèneamide et les sels cationiques d'amine quaternaires.

14. Formulation selon la revendication 13 dans laquelle le matériau polymérique est la polyvinyle pyrrolidone.

15. Formulation selon la revendication 14 dans laquelle la polyvinyle pyrrolidone a une masse moléculaire de 20 000 à 65 000.

16. Formulation selon la revendication 15 dans laquelle la polyvinyle pyrrolidone a une masse moléculaire de 45 000.

17. Formulation selon la revendication 10 qui contient facultativement un antioxydant choisi parmi le groupe comprenant le gallate de n-propyle, le BHA, le BHT et le monothioglycérol de 0,005 à 1,0% en poids/volume.

18. Formulation selon la revendication 10 qui contient éventuellement un glycol, un glycéride ou un glycol éther.

19. Formulation topique pour l'application directe sur la peau d'un animal pour le traitement efficace d'infestations parasitaires comprenant 5,0% en poids/volume de polyvinyle pyrrolidone, de masse moléculaire 45 000, 5,0% en poids/volume de 22,23-dihydro-13-O-[(2-méthoxyéthoxy)méthyl]avermectine B1 aglycone, 90% en volume/volume d'éthanol en quantité suffisante pour 100% avec de l'eau et 0,01% en poids/volume de BHT.

20. Procédé de préparation de la formulation selon la revendication 10 qui comprend les étapes consistant à dissoudre de 0,005 à 10% en poids/volume du composé d'avermectine selon la revendication 1 dans 1 à 95% en volume/volume d'éthanol pour former une solution claire, à ajouter et à dissoudre environ 0,005 à 1,0% en poids/volume de l'antioxydant et de 0,01 à 20% en poids/volume du matériau polymérique dans la solution, à ajouter jusqu'à 50% en volume/volume d'additif, à ajuster le volume à 100% par l'addition d'eau et à mélanger jusqu'à ce que la solution soit homogène.
